# EUROPÄISCHE PATENTSCHRIFT

(11) **EP 2 382 278 B1**
(45) Veröffentlichungstag und Bekanntmachung des Hinweises auf die Patenterteilung: **26.04.2017**
(21) Anmeldenummer: 09767968.2
(22) Anmeldetag: 02.12.2009
(51) Int. Cl.: C09K 3/18, C07C 29/88

(54) **VERFAHREN ZUR AUFARBEITUNG GEBRAUCHTER GLYKOLHALTIGER FLUGZEUGENTEISUNGSMITTEL**
PROCESSES FOR REGENERATION OF USED GLYCOL-CONTAINING AIRCRAFT DEICERS
PROCÉDÉS DE RÉGÉNÉRATION DE DÉGIVRANTS USÉS POUR AVIONS CONTENANT DES GLYCOLS

(30) Priorität: 24.12.2008 DE 102008063094
(43) Veröffentlichungstag der Anmeldung: 02.11.2011
(73) Patentinhaber: Clariant International Ltd, 4132 Muttenz (CH)
(72) Erfinder: KROPAC, Bernhard, 84556 Kasti (DE); KERBL, Wolfgang, 84549 Englsberg (DE); GATTER, Erich, 84556 Kasti (DE)
(74) Vertreter: Mikulecky, Klaus
(86) Internationale Anmeldenummer: PCT/EP2009/008583
(87) Internationale Veröffentlichungsnummer: WO 2010/072311

(56) Entgegenhaltungen:
- EP-A2- 0 637 620
- EP-A2- 1 889 658
- CA-A1- 2 116 827
- DE-A1- 19 650 682

## Beschreibung

Die Erfindung betrifft ein Verfahren zur Wiederaufbereitung von gebrauchten Flugzeugenteisungsmitteln auf der Basis von Glykolen.

Flugzeugenteisungsmittel auf Basis von Glykolen sind zum Beispiel in US-4 358 389 und US-4 744 913 beschrieben. Sie enthalten im Allgemeinen
(a) etwa 40 bis 80 Gew.-% mindestens eines Glykols mit 2 oder 3 Kohlenstoffatomen oder eines Diglykols mit 4 bis 6 Kohlenstoffatomen, zum Beispiel Ethylenglykol, Diethylenglykol, Propylenglykol und dergleichen,
(b) 0,05 bis 1,5 Gew.-% mindestens einer polymeren Komponente als Verdickungsmittel, zum Beispiel aus der Gruppe der Polyacrylate, Polymethacrylate, Xanthangummi und Cellulosederivate,
(c) 0,05 bis 1 Gew.-% mindestens eines Tensids, zum Beispiel Olefinsulfonate, Alkylarylsulfonate, Polyoxalkylate und dergleichen,
(d) mindestens einen Korrosionsinhibitor in einer wirksamen Menge, zum Beispiel aus der Gruppe der Triazole, Imidazole undloder Phosphorsäureester, und
(e) mindestens eine basischen Verbindung zur Einstellung des pH-Wertes von etwa 7,5 bis 11 und
(f) Wasser als Rest auf 100 Gew.-%.

Diese Flugzeugenteisungsmittel werden als solche (das heißt als Konzentrat) oder nach Verdünnung mit Wasser zu deren Konservierung und/oder zur Befreiung von Eis und/oder Schnee auf die zu behandelnden Flugzeugteile aufgebracht. Von den behandelten Flugzeugteilen fließt das Enteisungsmittel, das nun mit Schmelzwasser mehr oder weniger verdünnt und mit Sand, Gummiabrieb, Öl, Verbrennungsrückständen und dergleichen verunreinigt ist, in ein Sammelbecken und wird als Abwasser aus der Flugzeugenteisung oder als gebrauchtes Flugzeugenteisungsmittel bezeichnet.

Teilweise werden die gebrauchten Flugzeugenteisungsmittel mit Hilfe einer biologischen Kläranlage entsorgt. Dies führt aber - trotz der guten biologischen Abbaubarkeit von Glykolen - zu einer unerwünschten Belastung der Kläranlage, besonders bei niedrigen Außentemperaturen und damit verbundener reduzierter Bakterienaktivität, was beim Einsatz von Enteisungsmitteln im Allgemeinen der Fall ist. Ein weiterer Nachteil dieser Art der Entsorgung von Flugzeugenteisungsmitteln ist der Verlust des in großer Menge vorliegenden und wertvollen Glykols.

EP-A-0 637 620 offenbart ein Verfahren zur Wiederaufbereitung von gebrauchten Flugzeugenteisungsmitteln auf der Basis von Glykolen, in dem man
(1) das gebrauchte Flugzeugenteisungsmittel zunächst zur Abtrennung von den suspendierten Verunreinigungen filtriert,
(2) das im Schritt (1) erhaltene Filtrat zur Abtrennung von den polymeren Verdickungsmitteln einer Ultrafiltration unterwirft,
(3) das im Schritt (2) erhaltene Permeat zur Abtrennung von anwesenden Salzen und ionischen Verbindungen der Ultrafiltration mit einem Anionenaustauscher und einem Kationenaustauscher unterwirft, und
(4) die im Schritt (3) erhaltene Lösung zur Entfernung von überschüssigem Wasser und damit Einstellung des Glykolgehaltes auf den gewünschten Wert destilliert, und
(5) das erhaltene Glykol-Wassergemisch mit geeigneten Additiven für den Einsatz als Flugzeugenteisungsmittel nachadditiviert.

DE-A1-19650682 offenbart ein Verfahren zur Aufarbeitung von gebrauchten Flugzeugenteisungsmitteln, bei dem Glykole abdestilliert werden.

EP-A-1 889 658 offenbart ein Verfahren zur Aufarbeitung glykolhaltiger Flugzeugenteisungsmittel, bei dem diese unmittelbar einer Trennung mittels einer Membran unterworfen werden, wobei sich in einem späteren Verfahrensschritt eine Destillation ausschließt.

Es wurde nun beobachtet, dass die nach dem Stand der Technik aufgearbeiteten Enteisungsmittel nach einigen Recyclierungsschritten noch erhebliche Anteile von organischen Säuren und deren Glykolestern sowie anderen Verunreinigungen enthalten. Diese bewirken eine verminderte Langzeitstabilität neuer verdickter Enteisungsmittel, die mit derart aufgearbeiteten Enteisungsmittelbestandteilen hergestellt wurden. Dieses Problem ist durch eine einfache Destillation der gebrauchten Enteisungsmittel nicht zu lösen, da bei einer Destillation der im Enteisungsmittel enthaltene Verdicker ein Gel ergibt, das die Destillationsvorrichtung verstopft.

Die Aufgabe der Erfindung besteht demnach darin, ein einfaches und kostengünstiges Verfahren vorzuschlagen, das ein weitgehendes Wiedergewinnen und Wiedereinsetzen insbesondere der Glykole von gebrauchten Flugzeugenteisungsmitteln ermöglicht, und wobei die Wiederverwendbarkeit der Glykole erhalten bleibt.

Gegenstand vorliegender Erfindung ist somit gemäß Anspruch 1 ein Verfahren zur Aufarbeitung gebrauchter glykolhaltiger Flugzeugenteisungsmittel, indem man
(1) aus dem gebrauchten Flugzeugenteisungsmittel die darin suspendierten Verunreinigungen abtrennt,
(2) das so erhaltene Produkt in einer Weise behandelt, dass die darin enthaltenen Verdicker nicht mehr zur Gelbildung fähig sind,
(3) vor oder nach Schritt (2) einen pH > 7 einstellt,
(4) und danach die Glykole fraktionierend abdestilliert.

Bei den Glykolen handelt es sich in bevorzugter Ausführungsform um Glykole mit 2 oder 3 Kohlenstoffatomen oder Diglykole mit 4 bis 6 Kohlenstoffatomen, zum Beispiel Ethylenglykol, Diethylenglykol oder Propylenglykol. Besonders bevorzugt sind Propylenglykol und Monoethylenglykol.

Vorzugsweise werden in einem ersten Schritt die ungelöst vorliegenden Bestandteile abfiltriert. Das sind zum Beispiel Feststoffe wie Sand und Gummiabrieb, ferner Schwebstoffe, Ölpartikel und dergleichen. Zu dieser Grobfiltration, die vorzugsweise bei Raumtemperatur und Atmosphärendruck durchgeführt wird, können die üblichen Filtermaterialien eingesetzt werden, zum Beispiel Papier, Tuch, Gewebe und dergleichen.

Die Verhinderung der Gelbildung durch die im gebrauchten Flugzeugenteisungsmittel enthaltenen Verdicker kann auf verschiedene Weise erfolgen.

Das durch die Grobfiltration erhaltene Filtrat kann in einer ersten bevorzugten Ausführungsform einer Ultrafiltration durch eine Membran unterworfen werden, so dass das polymere Verdickungsmittel abgetrennt wird. Daneben werden auch zum Beispiel anwesendes emulgiertes Öl, höhere Kohlenwasserstoffe, Tenside und dergleichen in einem mehr oder weniger hohen Ausmaß abgetrennt. Der Ultrafiltrationsschritt wird bevorzugt in der Weise durchgeführt, dass eine Ultrafiltrationsmembran mit einer nominellen Trenngrenze (cut off) von 1.000 bis 500.000 eingesetzt wird, vorzugsweise mit einem cut off von 100.000 bis 300.000. Die Ultrafiltrationsmembran besteht vorzugsweise aus einem organischen Polymeren oder einem anorganischen Material, wobei Membrane aus Polysulfonen, Polyethersulfonen oder Polyaramiden bevorzugt sind. Die Membran ist vorzugsweise als Membranmodule Rohrmodule, Kapillarmodule, Plattenmodule und Spiralwickelmodul ausgeprägt. Die Ultrafiltration wird im Allgemeinen bei einem transmembranen Druck von 2 bis 10 bar, vorzugsweise 3 bis 6 bar durchgeführt. Die Temperatur beträgt von 20 bis 80 °C, vorzugsweise 40 bis 60 °C, um die zunehmende Viskosität des Konzentrats beim Anreichern des Verdickerpolymers während der Ultrafiltration herabzusetzen.

Die nach der Ultrafiltration erhaltene Flüssigkeit enthält im Wesentlichen Glykole und Wasser. Diese Glykol/Wasser-Mischung wird nach Alkalisierung auf pH > 7 einer fraktionierenden Destillation unterworfen, um die Glykole daraus abzutrennen.

In einer weiteren bevorzugten Ausführungsform wird das Produkt der Grobfiltration alkalisiert. Die Menge an Alkalien beträgt im Allgemeinen mindestens 0,1 Gew.-%, bezogen auf das Produkt der Grobfiltration. Bevorzugt ist eine Menge von 0,3 bis 3 Gew.-%, insbesondere 0,5 bis 2 Gew.-%. Geeignete alkalische Mittel sind beispielsweise Oxide und Hydroxide von Alkali- und Erdalkalimetallen, wie beispielsweise Calciumoxid, Calciumhydroxid, Natriumhydroxid oder Kaliumhydroxid. Die Alkalien können fest oder in Lösung, vorzugsweise wässriger Lösung verwendet werden. Das alkalisierte gebrauchte Flugzeugenteisungsmittel wird sodann einer ersten Destillationsstufe unterzogen, in der Wasser abgetrennt wird. Die vorstehende Verfahrensweise entspricht den oben angegebenen Verfahrensschritten (2) und (3),

Der Sumpf dieser ersten Destillationsstufe, der die Glykole enthält, wird sodann einer fraktionierenden Destillation unterworfen, bei der die Glykole als Destillat anfallen. Zur Destillation wird das Konzentrat, gegebenenfalls nach Einstellung des pH, in eine Vorlage gegeben, und über eine Destillationskolonne fraktionierend destilliert. Die Verfahren und Vorrichtungen zur Durchführung dieser Destillation können den im Stand der Technik bekannten entsprechen. Das aufbereitete Glykol wird als Destillat erhalten. Der Destillationssumpf wird verworfen.

Das erfindungsgemäße Verfahren ist besonders geeignet für gebrauchte Flugzeugenteisungsmittel, welche neben Wasser
(a) 1 bis 60 Gew.-%, vorzugsweise 5 bis 50 Gew.-% mindestens eines Glykols mit 2 oder 3 Kohlenstoffatomen oder eines Diglykols mit 4 bis 6 Kohlenstoffatomen, zum Beispiel Ethylenglykol, Diethylenglykol, Propylenglykol,
(b) bis zu 0,8 Gew.-%, vorzugsweise 0,05 bis 0,5 Gew.-%, von den genannten wasserlöslichen Polymeren aus der Gruppe der Polyacrylate und Polymethacrylate,
(c) etwa 0,01 bis 1 Gew.-% von den genannten Tensiden, vorzugsweise aus der Gruppe der anionischen Tenside, zum Beispiel Sulfonate wie Olefinsulfonate und Alkylbenzolsulfonate,
(d) etwa 0,001 bis 0,1 Gew.-% Korrosionsinhibitor, vorzugsweise aus der Gruppe der Triazole enthalten.

Die angegebene Zusammensetzung bezieht sich auf das Flugzeugenteisungsmittel vor seinem Gebrauch.

Die angestrebte Qualität der mit dem erfindungsgemäßen Verfahren gewonnenen Glykole wird durch folgende Kriterien festgelegt:
a) eine Tensidkonzentration von weniger als 100 ppm
b) ein Wassergehalt nach DIN 51 777 von max. 0,5 Gew.-%
c) ein Glykolsäuregehalt von weniger als 100 ppm, vorzugsweise weniger als 20 ppm
d) ein Ameisensäuregehalt von weniger als 100 ppm, vorzugsweise weniger als 20 ppm
e) ein Milchsäuregehalt von weniger als 100 ppm, vorzugsweise weniger als 20 ppm
f) ein Essigsäuregehalt von weniger als 100 ppm, vorzugsweise weniger als 20 ppm
g) ein Propionsäuregehalt von weniger als 100 ppm, vorzugsweise weniger als 20 ppm

Der angestrebte Gehalt an Glykolestern der unter c) bis g) genannten Säuren ist höchstens so groß wie der Gehalt an den Säuren.

## Patentansprüche

1. Verfahren zur Aufarbeitung gebrauchter glykolhaltiger Flugzeugenteisungsmittel, indem man
(1) aus dem gebrauchten Flugzeugenteisungsmittel die darin suspendierten Verunreinigungen abtrennt,
(2) das so erhaltene Produkt in einer Weise behandelt, dass die darin enthaltenen Verdicker nicht mehr zur Gelbildung fähig sind,
(3) vor oder nach Schritt (2) einen pH > 7 einstellt,
(4) und danach die Glykole fraktionierend abdestilliert.

2. Verfahren nach Anspruch 1, worin die Abtrennung der suspendierten Verunreinigungen in Schritt (1) durch Filtration erfolgt, die eine Grobfiltration, und eine nachfolgende Ultrafiltration durch eine Membran umfasst.

3. Verfahren nach Anspruch 1 und/oder 2, worin die pH-Einstellung des in Schritt (1) erhaltenen Filtrats mit Oxiden oder Hydroxiden von Alkali- und Erdalkalimetallen erfolgt.

4. Verfahren nach einem oder mehreren der Ansprüche 1 oder 3, worin das Produkt aus Schritt (1) alkalisiert und sodann einer ersten Destillationsstufe unterzogen wird, in der Wasser abgetrennt wird.

5. Verfahren nach Anspruch 4, worin die Alkalisierung mit mindestens 0,1 Gew.-% Alkalien, bezogen auf das Produkt aus Schritt (1) erfolgt.

6. Verfahren nach Anspruch 5, worin die Alkalisierung mit KOH durchgeführt wird.

7. Verfahren nach einem oder mehreren der Ansprüche 1 bis 6, worin das Flugzeugenteisungsmittel vor seinem Gebrauch neben Wasser
(a) 1 bis 60 Gew.-% mindestens eines Glykols mit 2 oder 3 Kohlenstoffatomen oder eines Diglykols mit 4 bis 6 Kohlenstoffatomen,
(b) bis zu 0,8 Gew.-% wasserlösliche Polymere aus der Gruppe der Polyacrylate und Polymethacrylate,
(c) 0,01 bis 1 Gew.-% Tenside,
(d) 0,001 bis 0,1 Gew.-% Korrosionsinhibitoren enthält.

## Claims

1. Process for regenerating used glycol-containing aircraft deicers by
(1) removing from the used aircraft deicer the impurities suspended therein,
(2) treating the product thus obtained in such a manner that the thickeners present therein are no longer capable of gel formation,
(3) adjusting to a pH > 7 before or after step (2),
(4) and subsequently fractionally distilling off the glycols.

2. Process according to Claim 1, wherein the suspended impurities in step (1) are removed by filtration, which involves a coarse filtration and a subsequent ultrafiltration through a membrane.

3. Process according to Claim 1 and/or 2, wherein the pH of the filtrate obtained in step (1) is adjusted using oxides or hydroxides of alkali metals and alkaline earth metals.

4. Process according to one or more of Claims 1 or 3, wherein the product from step (1) is made alkaline and is then subjected to a first distillation stage in which water is removed.

5. Process according to Claim 4, wherein the alkalization is effected using at least 0.1% by weight of alkalis, based on the product from step (1).

6. Process according to Claim 5, wherein the alkalization is carried out using KOH.

7. Process according to one or more of Claims 1 to 6, wherein the aircraft deicer comprises, prior to its use, besides water,
(a) 1 to 60% by weight of at least one glycol having 2 or 3 carbon atoms or of a diglycol having 4 to 6 carbon atoms,
(b) up to 0.8% by weight of water-soluble polymers from the group of polyacrylates and polymethacrylates,
(c) 0.01 to 1% by weight of surfactants,
(d) 0.001 to 0.1% by weight of corrosion inhibitors.

## Revendications

1. Procédé pour le traitement d'agents usagés de dégivrage d'avions, contenant du glycol, selon lequel
(1) on sépare les impuretés en suspension dans les agents usagés de dégivrage d'avions,
(2) on traite le produit ainsi obtenu de manière telle que les épaississants qui y sont contenus ne sont plus aptes à la formation de gel,
(3) on règle un pH > 7 avant ou après l'étape (2) et
(4) on élimine ensuite les glycols par distillation fractionnée.

2. Procédé selon la revendication 1, dans lequel la séparation des impuretés en suspension dans l'étape (1) est réalisée par filtration qui comprend une filtration grossière et une ultrafiltration consécutive à travers une membrane.

3. Procédé selon la revendication 1 et/ou 2, dans lequel le réglage du pH du filtrat obtenu dans l'étape (1) a lieu avec des oxydes ou des hydroxydes de métaux alcalins et de métaux alcalino-terreux.

4. Procédé selon l'une ou plusieurs des revendications 1 ou 3, dans lequel le produit de l'étape (1) est alcalinisé et ensuite soumis à une première étape de distillation dans laquelle on sépare de l'eau.

5. Procédé selon la revendication 4, dans lequel l'alcalinisation a lieu avec au moins 0,1% en poids d'alcalis par rapport au produit de l'étape (1).

6. Procédé selon la revendication 5, dans lequel l'alcalinisation est réalisée avec du KOH.

7. Procédé selon l'une ou plusieurs des revendications 1 à 6, dans lequel l'agent de dégivrage d'avions contient, avant son utilisation, outre de l'eau
(a) 1 à 60% en poids d'au moins un glycol comprenant 2 ou 3 atomes de carbone ou d'au moins un diglycol comprenant 4 à 6 atomes de carbone,
(b) jusqu'à 0,8% en poids en poids de polymères solubles dans l'eau du groupe des polyacrylates et des polyméthacrylates,
(c) 0,01 à 1% en poids de tensioactifs,
(d) 0,001 à 0,1% en poids d'inhibiteurs de corrosion.
